# EUROPEAN PATENT APPLICATION

(11) **EP 4 621 800 A1**
(43) Date of publication of application: **24.09.2025**
(21) Application number: 23891590.4
(22) Date of filing: 14.11.2023
(51) Int. Cl.: G16H 50/00

(54) **PREDICTION SYSTEM, PREDICTION DEVICE, PREDICTION METHOD, CONTROL PROGRAM, AND RECORDING MEDIUM**

(30) Priority: 14.11.2022 JP 2022182127
(71) Applicant: Kyocera Corporation, Kyoto-shi, Kyoto 612-8501 (JP); The University of Tokyo, Bunkyo-ku, Tokyo 113-8654 (JP)
(72) Inventor: WATANABE, Kenichi, Kyoto-shi, Kyoto 612-8501 (JP); HONDA, Shintaro, Kyoto-shi, Kyoto 612-8501 (JP); KAMEI, Kentaro, Kyoto-shi, Kyoto 612-8501 (JP); FUNAMOTO, Miho, Kyoto-shi, Kyoto 612-8501 (JP); MORO, Toru, Tokyo 113-8654 (JP); TANAKA, Sakae, Tokyo 113-8654 (JP)
(74) Representative: TBK
(86) International application number: PCT/JP2023/040998
(87) International publication number: WO 2024/106443

(57) **Abstract**

To accurately predict an effect of a drug when the drug is administered to a subject. A prediction system includes a storage that stores subject information regarding a bone of a subject, and a receiver that receives drug efficacy prediction information regarding an effect of a drug when the drug is administered to the subject. The subject information includes at least a medical image showing the bone of the subject. The prediction system generates drug efficacy prediction information based on a drug efficacy prediction model that predicts an effect of a drug having an action on bones based on the subject information and drug information regarding the drug.

## Description

### TECHNICAL FIELD

The present disclosure relates to a prediction system, a prediction device, a control method, and a control program, for predicting an effect of a drug that acts on bones.

### BACKGROUND OF INVENTION

Patent Document 1 discloses a technique for predicting an effect of an anticancer drug.

### CITATION LIST

### PATENT LITERATURE

Patent Document 1: JP 2022-013583 A

### SUMMARY

According to an aspect of the present disclosure, a prediction system includes a storage configured to store subject information regarding a bone of a subject, and a receiver configured to receive drug efficacy prediction information regarding an effect of a drug when the drug is administered to the subject, in which the subject information includes at least a medical image showing the bone of the subject, and the prediction system generates the drug efficacy prediction information based on a drug efficacy prediction model configured to predict an effect of a drug having an action on bones based on the subject information and drug information regarding the drug.

According to an aspect of the present disclosure, a prediction device includes an acquirer configured to acquire subject information regarding a bone of a subject, and a predictor configured to output drug efficacy prediction information regarding an effect of a drug when the drug is administered to the subject, in which the subject information includes at least a medical image showing the bone of the subject, and the predictor includes a drug efficacy prediction model configured to predict an effect of a drug having an action on bones based on the subject information and drug information regarding the drug.

According to another aspect of the present disclosure, a control method includes storing subject information regarding a bone of a subject, generating drug efficacy prediction information regarding an effect of a drug having an action on bones when the drug is administered to the subject based on a drug efficacy prediction model configured to predict the effect of the drug based on the subject information and drug information regarding the drug, and receiving the drug efficacy prediction information, in which the subject information includes at least a medical image showing the bone of the subject.

According to each aspect of the present disclosure, the prediction system may be implemented by one or more computers. In this case, the scope of the present disclosure also includes a control program of the prediction system that causes a computer to implement the prediction system by causing the computer to operate as each unit (software element) included in the prediction system, and a computer-readable recording medium recording the control program.

### BRIEF DESCRIPTION OF THE DRAWINGS

FIG. 1 is a diagram illustrating a configuration example of a prediction system according to an aspect of the present disclosure.
FIG. 2 is a diagram illustrating a configuration example of a prediction system according to another aspect of the present disclosure.
FIG. 3 is a block diagram illustrating an example of a configuration of a prediction device according to an aspect of the present disclosure.
FIG. 4 is a diagram illustrating an example of a configuration of a drug efficacy prediction model executed by a predictor.
FIG. 5 is a flowchart illustrating an example of a flow of training processing by a training unit.
FIG. 6 is a flowchart illustrating an example of a flow of processing executed by the prediction device.
FIG. 7 is a diagram illustrating an example of a medical service achieved by the prediction device.
FIG. 8 is a block diagram illustrating an example of a configuration of a prediction device according to another aspect of the present disclosure.
FIG. 9 is an example of a processed image including information regarding a basis for deriving changes in an image due to other factors.
FIG. 10 is an example of a browser image displaying analysis results in both cases where the changes in the image due to other factors are included and excluded.

### DESCRIPTION OF EMBODIMENTS

Drugs for improving a subject's bone condition include drugs that act on bone formation, drugs that act on bone resorption, and drugs that balance bone remodeling. These drugs are administered to the subject either alone or in combination.

According to an aspect of the present disclosure, an effect of a drug when administered to a subject can be accurately predicted.

The present disclosure will be described in detail below.

A subject's bone condition can be influenced by a balance between bone formation and bone resorption, lifestyle habits such as diet and exercise, genetics, and the like. For example, osteoporosis is a disease characterized by low bone mass and abnormalities in a microstructure of bone tissue, resulting in increased bone fragility and increased risk of fracture. Osteoporosis is often seen in elderly people and postmenopausal women. Osteoporosis is not limited to these people and may be caused by, for example, local circulatory disorders, calcium metabolic disorders, and the like.

When a subject's bone condition deteriorates and a fracture or the like occurs in a site important for motor function, surgery and hospitalization may be required. As a result, a quality of life (QOL) of the subject may be significantly reduced. In order to reduce the decline in the QOL of the subject, it is important to detect any tendency for the subject's bone condition to deteriorate as early as possible and to initiate intervention in an appropriate manner, including administration of appropriate drugs.

Embodiments according to the present disclosure will be described below. In the following description, a case in which a subject is a human (that is, a "human subject") is described as an example, but the subject is not limited to a human. The subject may be a mammal other than humans, such as Equidae, Felidae, Canidae, Bovidae, or Sus scrofa domesticus. The present disclosure also includes embodiments in which "human subject" is reworded as "animal" when the embodiments are applicable to any of these animals.

### First Embodiment

### Overview of Prediction Device 1

A prediction device 1 according to an aspect of the present disclosure outputs drug efficacy prediction information regarding an effect of a drug when the drug is administered to a human subject (hereinafter, may be referred to as a "subject") from subject information, which is information regarding the human subject, and drug information regarding the drug.

The subject information includes at least one selected from the group consisting of fracture risk factor information regarding a human subject's fracture risk factor, bone strength information regarding human subject's bone strength, information regarding a human subject's health condition, bone density information indicating a bone density of the human subject's bone, bone metabolic information indicating a bone metabolic state of the human subject's bone, and a medical image showing the human subject's bone. In the present disclosure, the human subject's bones may be multiple bones included in various sites of a body of the human subject, such as the chest, lower back, neck, hip joints, head, legs, arms, fingers, and feet. Alternatively, in the present disclosure, the human subject's bones may be specific bones of the human subject, such as the spine, thoracic vertebrae, lumbar vertebrae, pelvis, cervical vertebrae, skull, femur, fibula, tibia, radius, metacarpals, phalanges of the hand, calcaneus, metatarsals, and phalanges of the foot.

The subject information may include the fracture risk factor information regarding the human subject's fracture risk factor. The fracture risk factor information may be, for example, information used by the Fracture Risk Assessment Tool (FRAX (registered trade name)) or results thereof. The fracture risk factor information may include at least one selected from the information group A below.

(Information group A): Type (e.g., race), age, sex, weight, height, presence or absence of a fracture, fracture site, fracture history, fracture history of family members (e.g., parents), glucocorticoids, rheumatoid arthritis, secondary osteoporosis, underlying diseases (e.g., food and/or drug allergies, diseases related to and unrelated to the onset of osteoporosis), smoking history, drinking habits (e.g., drinking frequency and amount), occupational history, exercise history, medical history (e.g., history of bone disease), menstruation (e.g., cycle and presence or absence), menopause (e.g., likelihood and presence or absence), estimated time of menopause (e.g., estimated year and month, or year, month, and date), artificial joints (e.g., type and presence or absence of spinal implants or knee joints, and time of replacement surgery), blood test results, urine test results, drugs being taken, and gene sequence.

Information group A is information about the human subject that is useful for determining the bone density, bone metabolism, likelihood of fracture, balance between bone formation and bone resorption, and the like. Therefore, by using the subject information including at least one piece of information selected from the information group A, the prediction device 1 can more accurately predict an effect of a drug to be administered to the human subject.

Information regarding the presence or absence of a fracture may be detected based on a medical image showing the human subject. The fracture location may be information regarding a fracture site of the human subject that is estimated based on the medical image showing the human subject. Alternatively, the information regarding the presence or absence of a fracture may be information regarding a location of a bone in the human subject that is predicted to be more likely to suffer a fracture in the future.

Information regarding the blood test results may be, for example, information regarding the results of at least one selected from the group consisting of a biochemical test, a glucose metabolism test, and an endocrine system test.

The biochemical test may include, for example, information regarding test results of at least one selected from the blood test group B below.

(Blood test group B): Total protein (TP), albumin (ALB), aspartate aminotransferase (AST), alanine aminotransferase (ALT), lactate dehydrogenase (LDH), bone type ALP, creatinine (Cre), calcium (Ca), inorganic phosphorus (IP), total cholesterol (T-Cho), high density lipoprotein-cholesterol (HDL-C), triglycerides (TG), low density lipoprotein-cholesterol (calculated LDL-C), and C-reactive protein (CRP).

The glucose metabolism test may include, for example, information regarding test results of at least one selected from the glucose metabolism test group C below.

(Glucose metabolism test group C): Glucose (Glu), and hemoglobin A1c (HbA1c).

The endocrine system test may include, for example, information regarding test results of at least one selected from the endocrine system test group D below.

(Endocrine system test group D): Intact parathyroid hormone (intact PTH), 25-hydroxyvitamin D (25(OH)D), 1. 25-dihydroxyvitamin D (1.25(OH)2D), thyroid-stimulating hormone (YSH), CS, estradiol (E2), and free testosterone (free-TST).

The information regarding the blood test results may include, for example, information regarding test results of at least one selected from the urine test group E below.

(Urine test group E): Calcium (Ca), phosphorus (P), and creatinine (Cre).

Information regarding the drug being taken may include, for example, information such as a name of the drug, an amount being taken, and a period of time during which the drug is being taken. The information regarding the drug being taken may include information regarding a steroid drug being used.

The bone density information may be, for example, a measured value of bone density of a human subject's bone at a specific site. The bone density can be measured by dual-energy X-ray absorptiometry (DXA), ultrasonography, micro densitometry (MD), or the like. The bone density can be estimated from a plain X-ray image, a magnetic resonance imaging (MRI) image, a computed tomography (CT) image, an ultrasonic image, or a PET image, showing the subject's bone. DXA is a method of measuring a bone density using two different energy X-rays. DXA can generally be used to measure bone density in the lumbar vertebrae, proximal femur, and distal radius. In a DXA apparatus that measures a bone density using the DXA method, when the bone density of the lumbar vertebrae is measured, X-rays are emitted to the lumbar vertebrae of a human subject from the front of the lumbar vertebrae. In the DXA apparatus, when the bone density of the proximal femur is measured, X-rays are emitted to the proximal femur of the human subject from the front of the proximal femur. Ultrasonography is a method of measuring a bone density by applying ultrasonic waves to bones such as the lumbar vertebrae, femur, calcaneus, and tibia. Here, the "front of the lumbar vertebrae" and the "front of the proximal femur" are intended to be directions correctly facing capturing sites such as the lumbar vertebrae and the proximal femur, and may be the ventral side of the human subject's body or the back side of the human subject. The proximal femur includes, for example, at least one site selected from the group consisting of the neck, the trochanter, the diaphysis, and the entire proximal femur (neck, trochanter, and diaphysis). MD is a method of measuring a bone density by simultaneously imaging bones of hands and an aluminum plate using X-rays and comparing shades of the images captured. When measuring a bone density using MD, dorsal surfaces of both hands are irradiated with X-rays. A bone measured using MD is, for example, the second metacarpal of the hand. By using the bone density information indicating the bone density measured by DXA, which has high measurement accuracy, the accuracy of the drug efficacy prediction information output from the prediction device 1 can be improved. The bone density information may be information regarding bone quality, which will be described below, instead of the bone density, or may include information regarding bone quality in addition to the bone density.

The bone density information may be a bone density (estimated value) of a bone of a human subject estimated by the prediction device 1 from the medical image showing the bone of the human subject. Alternatively, the bone density information may be a future bone density (predicted value) of a bone of a human subject predicted by the prediction device 1 from the medical image showing the bone of the human subject. Thus, the prediction device 1 may estimate and/or predict a bone density of a bone of a human subject from a medical image showing the bone of the human subject. In this case, the prediction device 1 may be equipped with a trained bone density estimation/future prediction model that uses a medical image showing the human subject's bone as an explanatory variable and a measured value of bone density of the human subject's bone in the medical image as a response variable. The prediction device 1 may be equipped with a trained future bone density prediction model that uses a medical image showing the human subject's bone as an explanatory variable and a measured value of bone density of the human subject's bone a predetermined period of time after the medical image is taken as a response variable.

The prediction device 1 may calculate an estimated value and/or a predicted value of the bone density of the bone from an entire bone or a part of the bone in a medical image. When calculating the estimated value and the predicted value of the bone density of the bone from the part of the bone in the medical image, the prediction device 1 may calculate multiple estimated values and/or multiple predicted values for respective bone densities of the bone at multiple parts. When the prediction device 1 partially calculates the estimated value and the predicted value of the bone density of the bone, the prediction device 1 partially calculates the bone density at a region that mainly includes cancellous bone or partially calculates the bone density at a region that mainly includes cortical bone. Thus, an ability to calculate the estimated value and/or the predicted value of the bone density partially from the medical image facilitates prediction of vertebral or proximal femoral fractures. Not only the bone density, but also bone quality, which will be described later, can be calculated from an entire bone or a part of the bone in a medical image. As a method of focusing on the bone density or bone quality from a part of a medical image, for example, a trained segmentation method or the like can be used.

The bone density can be, for example, a value used in osteoporosis guidelines (such as, but not limited to, 2015 Prevention and Treatment Guidelines of the Japan Osteoporosis Society, General Incorporated Association, the same below). The bone density may be any value related to the density of bone, and a unique index can be used. To be specific, the bone density may be represented, for example, by at least one selected from the group consisting of bone mineral density per unit area (g/cm²), bone mineral density per unit volume (g/cm³), YAM (%), T-score, and Z-score. YAM (%) is an abbreviation for "Young Adult Mean" and is sometimes referred to as percentage of the young adult mean or percentage of the young mean. The bone density information may include information regarding a target location (e.g., the proximal femur and/or the vertebra) in addition to a measured value of bone density, and the estimated value of bone density and/or the predicted value of bone density.

Here, the prediction device 1 may use the bone density of the human subject's bone measured by a DXA apparatus, MD, ultrasonography, or the like instead of a medical image, as the bone density information. In this case, the bone density of the human subject measured within one year using, for example, the DXA apparatus can be used as the bone density information. Alternatively, the bone density information may be the future bone density of the bone of the human subject predicted by the prediction device 1 from the bone density measured by the DXA apparatus, and from the fracture risk factor information and/or the bone metabolic information.

The bone metabolic information may include at least one selected from the group consisting of bone formation marker information regarding osteoblasts, bone resorption marker information regarding osteoclasts, and bone matrix-related marker information regarding bone quality of the human subject. The bone metabolic information can be analyzed, for example, from blood or a urine sample of the human subject. The bone metabolic information can be, for example, data analyzed before drug treatment. The bone metabolic information may be, for example, data from a single analysis, or multiple data from analyses of samples collected multiple times. When multiple data are available, the bone metabolic information can be used as a rate of change, an average value, or a base value. The bone metabolic information can be, for example, data analyzed after drug treatment (e.g., three to six months after the start of drug administration) in addition to the data analyzed before drug treatment.

Here, the bone formation marker information may be information regarding alkaline phosphatase and/or procollagen type I N-propeptide, the bone resorption marker information may be information regarding at least one selected from the group consisting of deoxypyridinoline, type I collagen cross-linked N-telopeptide, type I collagen cross-linked C-telopeptide, and tartrate-resistant acid phosphatase-5b, and the bone matrix-related marker information may be information regarding at least one selected from the group consisting of undercarboxylated osteocalcin, pentosidine, and homocysteine.

The bone quality may be based on, for example, at least one selected from the group consisting of statistical properties of a bone, geometric properties of a bone, mechanical properties of a bone, and chemical properties of a bone. The bone quality may be based on, for example, at least one selected from the group consisting of bone metabolic markers, sex, race, whether or not menopause has occurred, age, cortical bone status, cancellous bone status, cancellous bone trabecular status, disease information, bone evaluation information, drug information, and presence or absence of a fracture. To be more specific, the bone quality can be measured using, for example, at least one selected from the group consisting of a bone formation marker, a bone resorption marker, a bone quality marker (e.g., vitamin K value), a cortical bone thickness, trabecular bone density, a trabecular bone orientation, and a trabecular bone score, but is not limited thereto. The trabecular bone score is an index calculated from raw data of a lumbar vertebrae DXA image (density grayscale image) taken from the front (anterior-posterior direction of the human subject).

The disease information may include, for example, at least one selected from the group consisting of osteoporosis, rheumatism, osteonecrosis (such as femoral head necrosis), systemic sclerosis, kidney disease, and osteopetrosis. The bone evaluation information may include information assessed by a fracture risk assessment tool. The drug information may include, for example, at least one selected from the group consisting of a trade name, a generic name, a dosage, an administration period, and an administration method (e.g., oral, intravenous injection, intramuscular injection, subcutaneous injection, etc.) for drugs including at least one selected from the group consisting of a drug that suppresses bone resorption, a drug that promotes bone formation, and other drugs (e.g., calcium preparations, vitamin preparations, female hormone preparations, etc.).

The bone quality may include, for example, the type of medullary cavity shape. The medullary cavity shape can be classified, for example, using the Dorr classification. The medullary cavity shape can be classified as follows using, for example, a thickness of the cortical bone and/or the shape of the medullary cavity.
Type A: A type with a thick cortical bone and a narrow and thin medullary cavity.
Type B: A type between Type A and Type C, with neither a narrow nor wide medullary cavity.
Type C: A type with a thin cortical bone and a wide medullary cavity.

The prediction device 1 can use any medical image with which a bone density of the bone, a bone shape, and a condition of tissues surrounding the bone (e.g., tendons, etc.) of the human subject to be predicted can be analyzed. Hence, the medical images are required to include, for example, at least one selected from the group consisting of a plain X-ray image, a computed tomography (CT) image, a magnetic resonance imaging (MRI) image, a PET image, and an ultrasonic image. When a plain X-ray image is used as a medical image, an imaging site for the plain X-ray image is not limited. A plain X-ray image used as a medical image is only required to show at least one selected from the group consisting of, for example, the head, neck, chest, lower back, hip joint, knee joint, ankle joint, foot, toe, shoulder joint, elbow joint, wrist joint, hand, fingers, and temporomandibular joint of the human subject. A plain radiographic image for estimation used to estimate the bone density, a site of the fracture, and the like of the human subject's bone, may be a front image showing a target site from the front, or may be a side image showing the target site from the side. When using a CT image as a medical image, for example, at least one selected from the group consisting of a three-dimensional image, a cross-sectional image perpendicular to a body axis connecting the head and legs (e.g., horizontal section), and a cross-sectional image parallel to the body axis (e.g., sagittal section or coronal section) can be used.

When the site to be analyzed is the hip joint (such as femoral neck and head) of the human subject, the prediction device 1 may use a plain X-ray image captured from a direction different from the front and side, such as an axial image and a Lauenstein image, as a medical image.

The subject information may include multiple pieces of time information respectively corresponding to multiple pieces of information included in the subject information. The time information may be a time or a point in time when multiple pieces of information included in the subject information are acquired, an examination date when the human subject is examined, a diagnosis date when the human subject is diagnosed, and a date when these pieces of information are entered to an electronic medical record, or the like.

The subject information may be information including the time information as described above, or may be information regarding the human subject at substantially the present time. "Substantially the present time" is intended to mean that a predetermined period of time is considered to be the same as the present time. Here, the predetermined period of time may be set as desired. For example, the predetermined period of time can be set to within the past three months, within the past six months, or within the past 12 months. To be more specific, even when a point in time when one piece of subject information is acquired and a point in time when another piece of subject information is acquired differ within a predetermined period, these points in time can be regarded as the same point in time. The subject information may be, for example, information regarding the human subject at a point in time when the bone density information and the bone metabolic information of the human subject are acquired, or at a point in time when a medical image of the human subject's bone is captured.

The subject information may be information regarding the human subject in the past or in the future, not in the present. For example, the subject information may be information regarding the human subject at a point in time when the bone density information and the bone metabolic information of the human subject have been previously acquired, or at a point in time when a medical image of the human subject's bone has been previously captured.

The drug information may include information regarding a drug having at least one selected from the group consisting of an action on bone formation, an action on bone resorption, and an action of balancing bone remodeling. The drug information may include at least a drug name and/or information regarding a mechanism of an action of the drug. By using such drug information, the prediction device 1 can more accurately predict an effect of the drug administered to the human subject. The drug information may include information regarding the chemical structural formula and physical property of the drug.

As the drug name, for example, at least one selected from the group consisting of a chemical name of an active ingredient, a generic name, a trade name, and a trivial name can be used. The drug name may be a development code of a drug manufacturer, a clinical trial institution and/or a medical institution. Drug names are not limited to any one of these, and may have multiple names. In this case, the usability of data can be improved. The information regarding the mechanism of an action may be, for example, biochemical information regarding how active ingredients contained in a drug act on target molecules, physiological and pharmacological information regarding important conditions for the active ingredients to act on the target molecules, or the like. Alternatively, the information regarding the mechanism of an action may include, for example, information regarding at least one selected from the group consisting of an action on bone formation, an action on bone resorption, and an action of balancing bone remodeling.

The drug information may include supporting information for each drug regarding a dosage form, a manufacturer, a lot number, a serial number, an administration method, administration conditions, an administration period, an administration interval, contraindication information, side effect information, a drug price, a commercial price, and countries where the drug is approved. The administration method may include, for example, oral administration or injection. The injection in the administration method may include information regarding an injection site, such as intravenous, intramuscular, intrathecal, or subcutaneous. When using the drug information that also includes these pieces of supporting information, the prediction device 1 may output the drug efficacy prediction information together with the supporting information for each drug, and present the drug efficacy prediction information and the supporting information to medical personnel. Alternatively, the prediction device 1 may output the drug efficacy prediction information regarding an effect of a drug when administered, for each piece of supporting information. Thus, the prediction device 1 can assist the medical personnel in determining a treatment plan including drug administration to the human subject. Here, the effect of a drug is an influence on a subject when the drug is administered to the subject, including the degree and/or presence or absence of the influence.

The drug efficacy prediction information may be fracture risk information indicating how the risk of a bone fracture in the human subject changes as a result of administration of a drug to the human subject. The fracture risk information in the drug efficacy prediction information includes, for example, information indicating that the fracture risk will be higher, the same, or lower. Alternatively, the drug efficacy prediction information may be a prediction result of future bone density of the human subject's bone. Alternatively, the drug efficacy prediction information may be a prediction result of current bone density of the human subject's bone. The drug efficacy prediction information may be information regarding whether the drug under consideration for administration to the human subject can improve a bone condition of the human subject. Alternatively, the drug efficacy prediction information may be information regarding the extent to which a drug under consideration for administration to the human subject can improve a bone condition of the human subject. The drug efficacy prediction information may be information indicating a bone condition of the human subject after administration of a drug to the human subject is continued for a predetermined period of time, or information indicating a change that occurs in the bone of the human subject before and after a predetermined period of time. Some drugs that act on a bone condition do not have an immediate effect, but rather develop an effect after a certain period of administration. According to the above configuration, the prediction device 1 can output the drug efficacy prediction information indicating an effect of a drug, even when the drug does not have an immediate effect.

The drug efficacy prediction information may be information regarding a drug able to change a bone condition of the subject when administration to the subject is continued for a predetermined period of time, or prediction information regarding a drug to be considered for administration to the human subject. **In** this case, the drug efficacy prediction information may include at least one selected from the group consisting of information regarding a drug that has an action on bone formation, information regarding a drug that has an action on bone resorption, and information regarding a drug that balances bone remodeling. When the drug efficacy prediction information includes two pieces of information regarding both drugs mentioned above, each drug included in the drug efficacy prediction information may be associated with effect information indicating whether the drug acts on bone formation or bone formation.

Examples of drugs that have an action on bone formation include, but are not limited to, teriparatide acetate and teriparatide (gene recombination). Examples of drugs that have an action on bone resorption include, but are not limited to, calcitonin preparations, bisphosphonate preparations, and anti-RANKL monoclonal antibodies. Examples of drugs that have an effect of balancing bone remodeling include active vitamin D3 preparations (e.g., calcitriol, eldecalcitol, alfacalcidol, etc.).

To be more specific, the drug efficacy prediction information may be information indicating the degree of improvement of the human subject with a specific drug expressed as a percentage, or may be information indicating an improvement ratio of bone density expressed as a percentage.

The drug efficacy prediction information may include, for example, multiple pieces of prediction information regarding drug efficacy corresponding to multiple administration periods during which the drug is administered to the human subject. To be specific, the drug efficacy prediction information may include two or more pieces of prediction information regarding the drug efficacy for administration periods during which the drug is administered to the human subject, such as three months, six months, one year, and three years. Alternatively, when the drug efficacy prediction information includes multiple pieces of prediction information for the corresponding multiple drugs, appropriate administration periods for the multiple drugs may differ from one another. Alternatively, the drug efficacy prediction information may be prediction information regarding drug efficacy at a time when a classification class defined by guidelines or the like changes (e.g., when transitioning from an "osteoporosis" class to a "low bone mass" class).

The drug efficacy prediction information may include a prediction image indicating a prediction result regarding how the administered drug will change the human subject's bone. For such drug efficacy prediction information, the subject information including a medical image showing the human subject's bone is used. The prediction image may indicate not only a prediction result indicating a change in the human subject's bone but also a prediction result indicating an influence on tissues or the like surrounding the bone. Multiple prediction images may be displayed corresponding to administration periods that have elapsed, or the prediction images may change like a moving image within one image region. Displaying multiple prediction images may be, for example, displaying prediction images corresponding to multiple administration periods of time in parallel.

The drug efficacy prediction information may include side effect prediction information regarding a side effect predicted when the drug is administered to the human subject. The side effect prediction information may be information regarding at least one side effect from the following group of side effects, for example.

(Group of side effects) Fatigue, allergic reaction, anaphylactic shock, headache, vomiting, gastrointestinal disorder, constipation, vascular disorder, heartburn, kidney disorder, hepatic disorder, jaundice, hypercalcemia, hypocalcemia, osteonecrosis of the jaw, osteomyelitis of the jaw, osteonecrosis of the external auditory canal, urolithiasis, convulsions, palpitations, loss of appetite, and hyperuricemia.

The side effect prediction information may include prediction information regarding a specific side effect that may occur in the human subject administered the drug, or may include information indicating a likelihood (such as probability of occurrence) that one or more side effects may occur in the human subject. The side effect prediction information may include multiple pieces of prediction information, each of which relates to the occurrence of a side effect depending on an administration period of time during which the drug is administered to the human subject. The side effect prediction information may further include prediction information that predicts how the probability of occurrence of the side effect in the human subject changes due to ingestion of another drug different from the drug being predicted, diet, or daily activities. For example, the side effect prediction information may include prediction information indicating how the probability of occurrence of the side effect in the human subject changes when a drug different from the drug being predicted is taken to an extent (e.g., duration and amount of intake).

When the drug is administered to the human subject, the prediction device 1 outputs the drug efficacy prediction information that accurately predicts an effect of the drug. Thus, the prediction device 1 can present to the human subject, the medical personnel in charge of the subject, and the like how the bone condition of the human subject may change as a result of the administration of the drug before the drug is administered.

### Configuration of Prediction System 100a

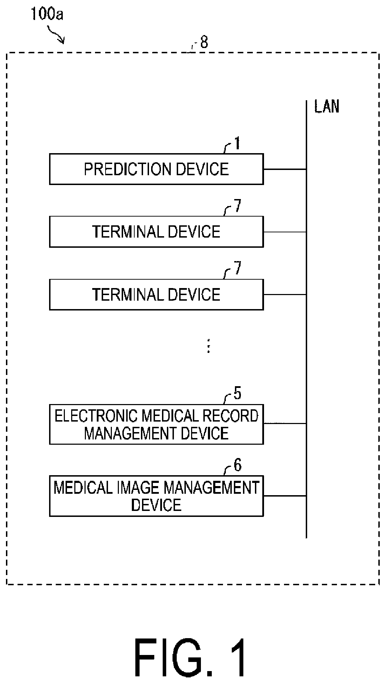

First, in an aspect of the present disclosure, a configuration of a prediction system 100a will be described with reference to FIG. 1. FIG. 1 is a diagram illustrating an example configuration of the prediction system 100a in a medical facility 8 in which a prediction device 1 is installed. In the following, the prediction device 1 that acquires, as subject information, a medical image showing a human subject's bone will be described as an example.

The prediction system 100a includes the prediction device 1 and one or more terminal devices 7 communicably connected to the prediction device 1. The prediction device 1 is a computer that outputs drug efficacy prediction information from a medical image showing a human subject's bone and transmits the drug efficacy prediction information to the terminal devices 7. The prediction device 1 may be a cloud-based device or may be an on-premises device installed in the medical facility 8 or a company that provides analysis services.

The terminal device 7 (receiver) functions as a receiver that receives the drug efficacy prediction information in the prediction system 100a. That is, the terminal device 7 receives the drug efficacy prediction information from the prediction device 1 and presents the drug efficacy prediction information. The terminal device 7 is a computer used by medical personnel such as a doctor belonging to the medical facility 8. The terminal device 7 is, for example, a personal computer, a tablet terminal, or a smartphone. The terminal device 7 includes a communicator for transmitting and receiving data to and from another device, input units such as a keyboard and a microphone, a display capable of displaying information included in the drug efficacy prediction information, and an output unit such as a speaker. An example is illustrated in which a local area network (LAN) is provided and the prediction device 1 and the terminal devices 7 are connected to the LAN in the medical facility 8 illustrated in FIG. 1, but the network is not limited thereto. For example, the network in the medical facility 8 may be the Internet, a telephone communication line network, an optical fiber communication network, a cable communication network, a satellite communication network, or the like.

In addition to the prediction device 1 and the terminal devices 7, a medical image management device 6 (storage) that can function as a storage that stores the subject information in the prediction system 100a may be communicably connected to the LAN in the medical facility 8. The medical image management device 6 is a computer that functions as a server for managing medical images captured in the medical facility 8. In this case, the prediction device 1 may acquire a medical image showing the human subject's bone from the medical image management device 6.

In the prediction system 100a, an electronic medical record management device 5 (storage) that can function as a storage that stores the subject information may be communicably connected to the LAN in the medical facility 8. The electronic medical record management device 5 is a computer that functions as a server for managing various kinds of subject information regarding the human subject. In this case, the prediction device 1 may acquire, from the electronic medical record management device 5, the various kinds of subject information regarding the human subject.

The LAN in the medical facility 8 may be communicably connected to an external communication network. In the medical facility 8, the prediction device 1 and the terminal devices 7 may be directly connected without going through a LAN.

### Configuration of Prediction Device 1

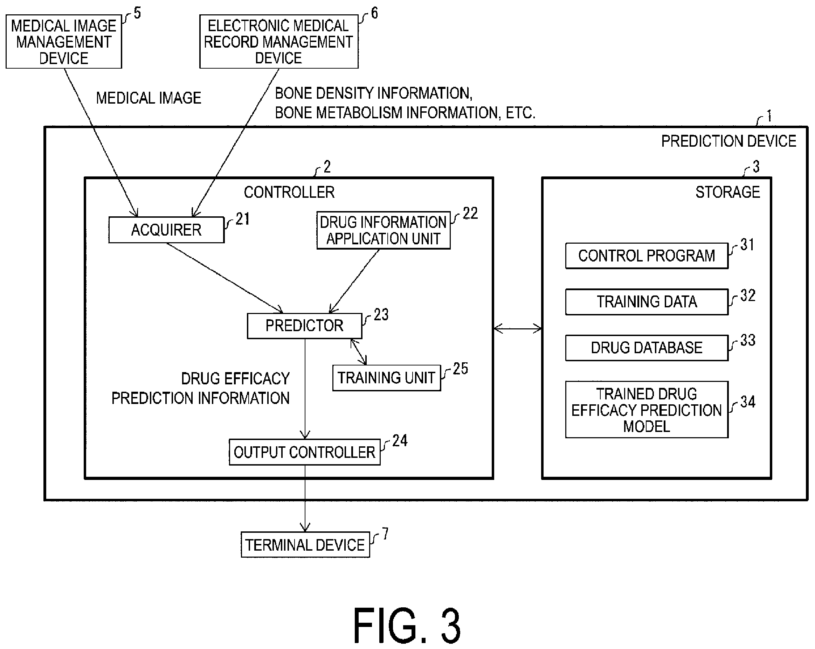

A configuration of the prediction device 1 applied to the prediction system 100a illustrated in FIG. 1 will be described with reference to FIG. 3. FIG. 3 is a block diagram illustrating an example of a configuration example of the prediction device 1.

The prediction device 1 includes a controller 2 that collectively controls units in the prediction device 1, and a storage 3 that stores various data to be used by the controller 2. The controller 2 includes an acquirer 21, a drug information application unit 22, a predictor 23, an output controller 24, and a training unit 25. The storage 3 stores a control program 31, which is a program for performing various controls in the prediction device 1, as well as training data 32, a drug database 33, and a trained drug efficacy prediction model 34 (drug efficacy prediction model). In an example, the subject information may be stored in the storage 3.

### Acquirer 21

The acquirer 21 acquires the subject information including at least one selected from the group consisting of risk factor information regarding a fracture risk factor of the human subject, bone density information indicating a bone density of a subject, bone metabolic information indicating a bone metabolic state of the subject, and a medical image showing a bone of the subject. For example, the acquirer 21 illustrated in FIG. 3 may be capable of acquiring, from the electronic medical record management device 5, the risk factor information regarding the fracture risk factor of the subject, the bone density information indicating the bone density of the human subject, and the bone metabolic information indicating the bone metabolic state. The acquirer 21 illustrated in FIG. 3 may be capable of acquiring, from the medical image management device 6, the medical image, which is an image showing the human subject's bone. The subject information is input data input to the predictor 23.

### Drug Information Application Unit 22

The drug information application unit 22 acquires drug information regarding at least one selected from the group consisting of information regarding a drug that has an action on bone formation, information regarding a drug that has an action on bone resorption, and information regarding a drug that has an action of balancing bone remodeling. For example, as illustrated in FIG. 3, the drug information application unit 22 may read the drug database 33 in the storage 3 to acquire the drug information. Alternatively, the drug information application unit 22 may acquire the drug information from an external drug information management device via a network such as the Internet. The drug information can be input data input to the predictor 23.

### Predictor 23

The predictor 23 inputs the subject information into the trained drug efficacy prediction model 34 that predicts an effect of a drug based on the drug information regarding the drug having an action on bones, thereby generating and outputting the drug efficacy prediction information regarding the effect of the drug when the drug is administered to the human subject. The predictor 23 may output, as the drug efficacy prediction information, a prediction result indicating a difference between a future bone density and a current bone density of the human subject's bone, and/or a prediction result indicating a change in the bone density of the human subject's bone from the current bone density.

For example, when the medical image, the bone metabolic information, and the like of the human subject are input as the subject information, the predictor 23 may output a list of drugs to be considered for administration to the human subject, and drug efficacy information (described later) regarding effects of various drugs included in the list, even when drug information regarding the drugs to be administered to the human subject is not input. In other words, input of the drug information is not essential for the predictor 23 to output the drug efficacy information.

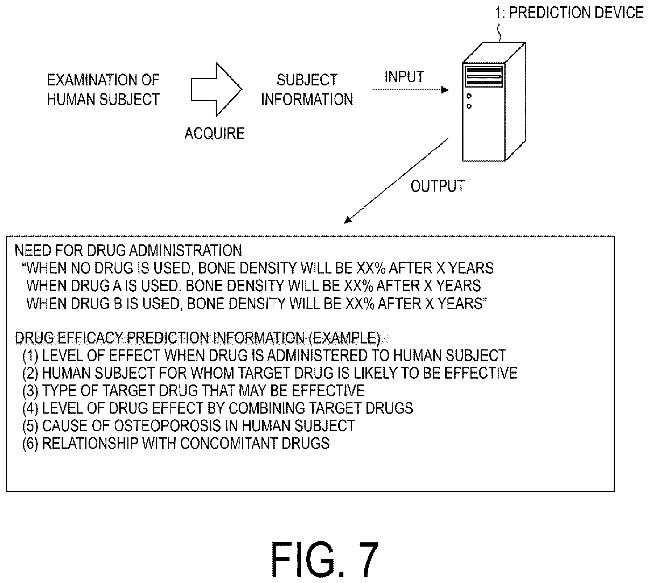

The predictor 23 may output a prediction result regarding a site of the fracture in the human subject. Alternatively, the predictor 23 may output a prediction result indicating a likelihood that the human subject will suffer a fracture in the future. Specific examples of information output from the predictor 23 will be described later (see FIG. 7).

The trained drug efficacy prediction model 34 may be trained using the training data 32 in which multiple pieces of examinee information regarding multiple examinees administered the drug and the drug information regarding the drug administered to each of the multiple examinees are used as explanatory variables, and effects of the drug on the multiple examinees are used as response variables. The trained drug efficacy prediction model 34 is, for example, a model obtained as a result of training processing performed by the training unit 25, which will be described later, on an untrained neural network. The examinee is required to be of the same biological species as the subject. In the following, an example will be described in which the examinee is a human.

The predictor 23 may output a prediction result when the drug is administered, and a prediction result when the drug is not administered. In this case, the trained drug efficacy prediction model 34 may be trained using the training data 32 in which multiple pieces of examinee information for multiple examinees to whom the drugs are administered and multiple pieces of examinee information for multiple examinees to whom the drugs are not administered are used as explanatory variables, and an effect of the drug on each of the examinees is used as a response variable.

The predictor 23 may output prediction results when different types of drugs are administered. In this case, the trained drug efficacy prediction model 34 may be trained using the training data 32 in which multiple pieces of examinee information regarding multiple examinees to whom drug A is administered and multiple pieces of examinee information regarding multiple examinees to whom drug B, which is a different type from drug A, is administered are used as explanatory variables, and effects of the drugs on the examinees are used as response variables.

The predictor 23 may output prediction results when different drugs are administered in combination. In this case, the trained drug efficacy prediction model 34 may be trained using the training data 32 in which multiple pieces of examinee information regarding multiple examinees administered drugs in different combinations are used as explanatory variables, and effects of the drugs on the multiple pieces of examinees are used as response variables.

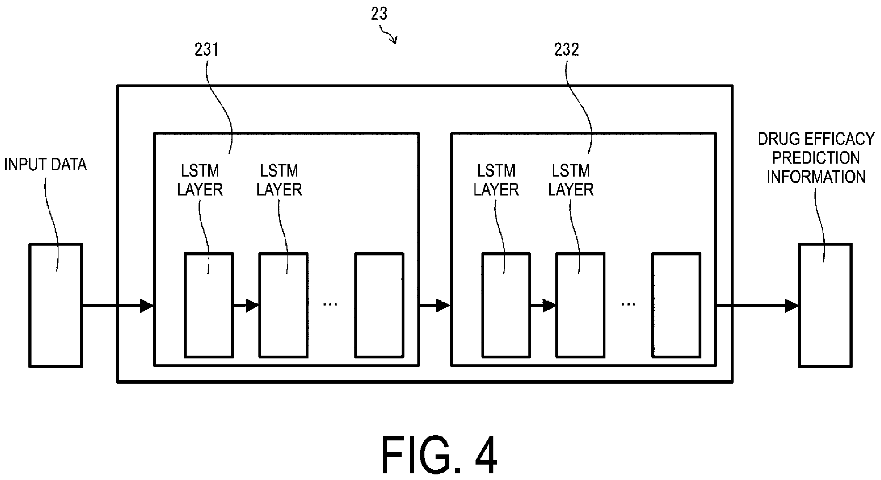

The predictor 23, in response to the subject information (e.g., medical image) and the drug efficacy information being input to an input layer 231 (see FIG. 4), executes operations based on the trained drug efficacy prediction model and outputs the drug efficacy prediction information from an output layer 232 (see FIG. 4). As an example, the predictor 23 may extract features from the subject information and the drug efficacy information, and use these features as input data. Known algorithms such as those listed below may be applied to extract these features.
- Convolutional neural network (CNN).
- Autoencoder.
- Recurrent neural network (RNN).
- Long short-term memory (LSTM).
- Convolutional long short-term memory (ConvLSTM).

The trained drug efficacy prediction model 34 is an operation model used by the predictor 23 when executing operations based on the input data. The trained drug efficacy prediction model 34 is generated by performing machine training on an untrained neural network using the training data 32, which will be described later. The training data 32, a configuration of the neural network, and specific examples of the training processing will be described later.

### Output Controller 24

The output controller 24 transmits the drug efficacy prediction information output from the predictor 23 to the terminal device 7. The prediction device 1 may include a display (not illustrated). In that case, the output controller 24 causes the display to display the drug efficacy prediction information.

### Training Unit 25

The training unit 25 controls training processing for the untrained neural network. The training unit 25 executes the training processing for the untrained neural network to create a neural network that functions as the predictor 23 from the untrained neural network. The training data 32 (described later) is used for this training. Specific examples of training executed by the training unit 25 will be described later.

### Training Data 32

The training data 32 is data including explanatory variables and response variables used in machine training to generate the trained drug efficacy prediction model 34 from the untrained neural network. In the training data 32, multiple pieces of examinee information regarding multiple examinees administered the drug and the drug information regarding the drug administered to each of the examinees are explanatory variables, and effects of the drug on the examinees are response variables.

Here, the examinee information is information that includes at least one selected from the group consisting of risk factor information regarding a fracture risk factor of the examinee, bone density information indicating a bone density of the examinee, bone metabolic information indicating a bone metabolic state of the examinee, and a medical image showing an examinee's bone. The examinee information may include the risk factor information regarding the fracture risk factor of the examinee. The fracture risk factor information may include at least one piece of information selected from the information group A described above. Here, the bone density information may be a measured value of bone density of the examinee at a specified site. The bone density may be measured by dual-energy X-ray absorptiometry (DXA), ultrasonography, micro densitometry (MD), or the like. Alternatively, the bone density may be estimated from a plain X-ray image, a magnetic resonance imaging (MRI) image, a positron emission tomography (PET) image, or a computed tomography (CT) image showing the human subject's bone.

Effects of the drugs on the examinees may be levels at which the drugs administered to the examinees improve bone conditions of the examinees. Alternatively, the effects of the drugs on the examinees may be information indicating bone conditions of the examinees after the drugs are administered continuously for a predetermined period of time, or changes in bone conditions before and after the drugs are administered continuously for a predetermined period of time.

### Configuration of Predictor 23

A configuration of the predictor 23 will be described below with reference to FIG. 4. The configuration illustrated in FIG. 4 is an example, and the configuration of the predictor 23 is not limited to this.

As illustrated in FIG. 4, the predictor 23 executes operations based on the trained drug efficacy prediction model 34 on input data input to the input layer 231, and outputs the drug efficacy prediction information from the output layer 232.

In FIG. 4, the predictor 23 includes a neural network including the input layer 231 and the output layer 232. The neural network is a neural network suitable for handling time series information. The neural network is, for example, an LSTM. The neural network may be any neural network suitable for handling time series information and position information in combination. The neural network is, for example, a ConvLSTM network, which combines CNN and LSTM. The input layer 231 can extract features of time changes in the input data. The output layer 232 can calculate new features based on the features extracted in the input layer 231 and the time changes and initial values of the input data. Each of the input layer 231 and the output layer 232 includes multiple LSTM layers. Each of the input layer 231 and the output layer 232 may include three or more LSTM layers.

### Training Processing by Training Unit 25

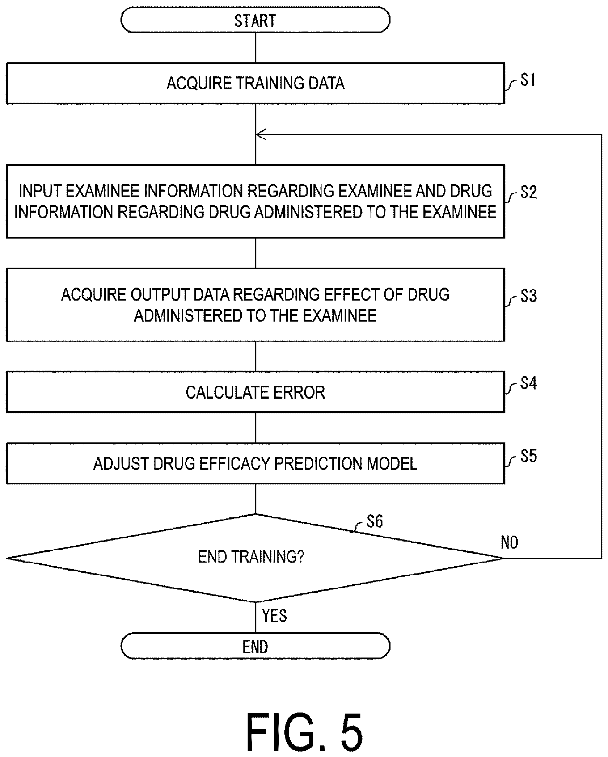

The training processing for generating the trained drug efficacy prediction model 34 will be described below with reference to FIG. 5. FIG. 5 is a flowchart illustrating an example of a flow of training processing by the training unit 25.

The training unit 25 acquires the training data 32 from the storage 3 (step S1). The training data 32 includes explanatory variables to be input to the input layer 231. Here, the explanatory variables are multiple pieces of examinee information regarding multiple examinees administered the drug and the drug information regarding the drug administered to each of the examinees.

Subsequently, the training unit 25 inputs examinee information regarding a certain examinee A and drug information regarding a drug administered to the examinee A to the input layer 231 (step S2).

Subsequently, the training unit 25 acquires output data regarding an effect of the drug administered to the examinee A from the output layer 232 (step S3). This output data includes the same contents as the response variables in the training data 32. In FIG. 5, the order of step S2 and step S3 may be reversed. Alternatively, in FIG. 5, step S2 and step S3 may be executed simultaneously.

Subsequently, the training unit 25 acquires response variables regarding the examinee A included in the training data 32. Then, the training unit 25 compares the output data acquired in step S3 with the response variables regarding the examinee A, calculates an error (step S4), and adjusts the drug efficacy prediction model being trained so as to reduce the error (step S5).

Any known method is applicable to adjust the drug efficacy prediction model being trained. For example, backpropagation may be used as a method for adjusting the drug efficacy prediction model. The adjusted drug efficacy prediction model becomes a new drug efficacy prediction model, and in subsequent operations, the predictor 23 uses the new drug efficacy prediction model. During an adjustment stage of the drug efficacy prediction model, parameters used in the predictor 23 (e.g., filter coefficients, weighting factors, etc.) may be adjusted.

If the error is not within the specified range, and if explanatory variables for all examinees included in the training data 32 have not been input (NO in step S6), the training unit 25 returns to step S2 and repeats the training processing. When the error is within the specified range, and when explanatory variables for all examinees included in the training data 32 have been input (YES in step S6), the training unit 25 ends the training processing.

When the training processing as described above is adopted, the predictor 23 can output drug efficacy prediction information regarding an effect of the drug to be administered to the human subject from the subject information and the drug information. In addition, when the training processing as described above is adopted, the predictor 23 can output drug efficacy prediction information regarding one or more drugs that are candidates for administration to the human subject from the subject information.

### Other Embodiments

The training data 32 may include multiple pieces of information regarding combinations of multiple types of drugs administered to examinees as explanatory variables, and may include multiple pieces of information regarding effects of the multiple types of drugs on the examinees as response variables. When training processing using such training data 32 is adopted, the predictor 23 can output the drug efficacy prediction information for each of the combinations of the multiple types of drugs to be administered to a human subject.

### Processing Executed by Prediction Device 1

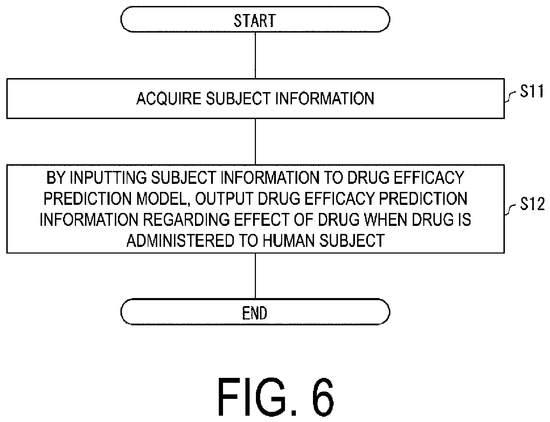

A flow of processing executed by the prediction device 1 will be described below with reference to FIG. 6. FIG. 6 is a flowchart illustrating an example of the flow of processing executed by the prediction device 1. FIG. 6 illustrates an example of processing for outputting the drug efficacy prediction information from subject information.

The subject information regarding a subject's bone is stored in the medical image management device 6, the electronic medical record management device 5, and the like (storing step). First, the acquirer 21 acquires the subject information (step S11: acquiring step) and inputs the subject information to the predictor 23.

The predictor 23 inputs the subject information into the trained drug efficacy prediction model 34, thereby generating and outputting drug efficacy prediction information regarding an effect of a drug when the drug is administered to the human subject (step S12: generating step and predicting step). The drug efficacy prediction information output from the predictor 23 may be transmitted to the terminal device 7. In this case, the terminal device 7 receives the drug efficacy prediction information (receiving step).

In step S12, drug information regarding a drug having an action on bone formation and/or an action on bone resorption may be input into the trained drug efficacy prediction model 34 together with the subject information.

### Medical Service Implemented by Prediction Device 1

FIG. 7 is a diagram illustrating an example of a medical service implemented by the prediction device 1.

When a human subject is examined in the medical facility 8 based on results of a periodical health check or subjective symptoms such as a suspected fracture, subject information regarding the human subject may be collected by a user (e.g., medical personnel) during a medical examination. The subject information is information that includes at least one selected from the group consisting of bone density information indicating a bone density of an acquired bone, bone metabolic information indicating a bone metabolic state of the bone of the human subject, and a medical image showing the bone of the human subject.

When the user inputs the acquired subject information into the prediction device 1, the prediction device 1 outputs drug efficacy prediction information regarding a drug (and/or a candidate drug) to be administered to the human subject. Alternatively, the user may input the acquired subject information and drug information regarding the drug (and/or the candidate drug) to be administered to the human subject to the prediction device 1.

The prediction device 1 may output, as drug efficacy prediction information, a bone density in the future when the human subject is not administered the drug and a bone density in the future when the human subject is administered the drug. Here, the output bone density may be represented by at least one selected from the group consisting of bone mineral density per unit area (g/cm²), bone mineral density per unit volume (g/cm³), YAM (%), T-score, and Z-score. YAM (%) is an abbreviation for "Young Adult Mean" and is sometimes referred to as percentage of the young adult mean or percentage of the young mean. For example, the output layer 230 may output the bone density represented by the bone mineral density per unit area (g/cm²) and the bone density represented by YAM, or may output the bone density represented by YAM, the bone density represented by T-score, and the bone density represented by Z-score. The output bone density may be categorized such that a T-score of - 1.0 or greater is displayed as a normal bone density, a T-score of -1.0 to -2.5 is displayed as a low bone density or osteopenia, and a T score of -2.5 or less is displayed as osteoporosis. Alternatively, the output bone density may be represented by a YAM ratio. The YAM ratio is a value indicating a diagnostic criterion, and may be categorized as "normal" when the YAM ratio is in a range of 100% to 80%, "low bone mass" when the YAM ratio is in a range of 80% to 70%, and "osteoporosis" when the YAM ratio is lower than 70%.

FIG. 7 illustrates the drug efficacy prediction information, such as "When nothing is done, bone density will be XX% after X years", "When drug A is used, bone density will be XX% after X years", and "When drug B is used, bone density will be XX% after X years". A user who has obtained such drug efficacy prediction information can appropriately consider and decide whether the drug should be administered to the human subject and which is more preferable to administer, drug A or drug B, based on the drug efficacy prediction information. As the drug efficacy prediction information in FIG. 7, the prediction device 1 may present a probability of future fracture instead of the bone density, or may present a probability of future fracture in addition to the bone density.

FIG. 7 illustrates only an example. For example, the drug efficacy prediction information may include drug efficacy prediction information such as "When drug A and drug B are used in combination, bone density will be XX% after X years" and "When drug A, drug B, drug C, ··· are used in combination, bone density will be XX% after X years". In addition, the drug efficacy prediction information may include drug efficacy prediction information such as "When drug A is administered P mg/day, bone density will be XX% after X years" and "When drug A is administered Q mg/day, bone density will be XX% after X years". Since some drugs have a limited administration period, the drug efficacy prediction information may include drug prediction information such as "When drug A is administered for the first K years and drug B is administered thereafter, bone density will be XX% after X years". Instead of the bone density, the drug prediction information may include information regarding a fracture risk, such as "a likelihood of developing a fracture in M years is XX%".

The prediction device 1 may be capable of outputting six pieces of drug efficacy prediction information indicated by (1) to (6) illustrated in FIG. 7. In FIG. 7, "target drug" refers to one or more drugs and/or candidate drugs to be administered to a human subject.
(1) The level of effect of the drug when administered to the human subject.
(2) Information regarding whether the human subject is a human subject who is expected to benefit from the target drug.
(3) The type of target drug that may be effective.
(4) The level of drug effect by combining the target drugs.
(5) The cause of osteoporosis in the human subject.
(6) The relationship with concomitant drugs.
For example, the drug efficacy prediction information described in (5) may be information indicating whether the cause is bone formation alone, bone resorption alone, or both bone formation and bone resorption.

When various additional pieces of information regarding a human subject are input, the prediction device 1 may output the type of drug suitable for the human subject.

For a human subject having a significantly low bone mass, a human subject who has already suffered a fracture, and a human subject at high risk of fracture, the prediction device 1 may output the type of drug that is highly effective in increasing the bone density. Examples of drugs that are highly effective in increasing the bone density include parathyroid hormone drugs. Here, the bone mass information may be information measured by a bone density measurement device such as DXA, or may be information obtained by estimating a bone density from an X-ray image using a trained parameter. For the fracture risk, information on a fracture risk factor from FRAX (registered trademark) may be used, information predicted from an X-ray image using a trained parameter may be used, or a combination thereof may be used. Bone mass is the sum of bone mineral and bone matrix protein. In the present disclosure, bone mass is an index related to bone density, and the bone mass is an amount of bone tissue in the skeleton.

The prediction device 1 may output drug efficacy prediction information according to the type of drug that is effective in reducing fractures. Such drug efficacy prediction is useful, for example, for human subjects at high risk of fracture. Examples of drugs that are effective in reducing fractures include bisphosphonate preparations and denosumab. For example, for a human subject who is at a high risk of fracture at a specific site, the prediction device 1 may extract the type of drug effective for fractures at the specific site, and output drug efficacy prediction information corresponding to the drug. To be more specific, for example, when the specific site is the proximal femur, the prediction device 1 may extract the type of drug that is effective in healing a fracture in the proximal femur, and output drug efficacy prediction information corresponding to the drug. The specific site may include, but is not limited to, the vertebral body and/or the proximal femur. Predicting a fracture at the specific site may be based, for example, in part, on estimating or predicting a bone density and/or bone quality in a region that primarily includes cancellous bone and/or cortical bone.

To be more specific, when predicting a bone density and/or fracture of the vertebra as the drug efficacy prediction information, the prediction device 1 may partially focus on a region of the vertebra that mainly contains cancellous bone. When predicting a bone density and/or fracture in the proximal femur as the drug efficacy prediction information, the prediction device 1 may partially focus on a region of the femur that mainly contains cortical bone.

For a human subject who is suspected to have increased bone resorption based on the measurement of bone metabolic markers, the prediction device 1 may output the type of drug that corresponds to a bone resorption inhibitor. Examples of drugs that fall under the category of bone resorption inhibitors include calcitonin drugs, bisphosphonate preparations, anti-RANKL antibodies, selective estrogen receptor modulator (SERM) preparations, and female hormone preparations.

For a human subject who is considered to have decreased bone formation, the prediction device 1 may output a type of drug that corresponds to a bone formation promoting drug. Examples of drugs that fall under the category of bone formation promoting drugs include active vitamin D3, parathyroid hormone drugs, and vitamin K2 preparations.

The information regarding drugs included in the drug efficacy prediction information output by the prediction device 1 may be selected based on information regarding an administration period, swallowability, and cost. Here, the administration period may include at least one piece of information regarding the age and menopause of the human subject. For a human subject who needs to take the drug for a long period of time, the prediction device 1 may output a type of drug that is suitable for long-term administration. Examples of drugs suitable for long-term administration include SERM preparations.

For a human subject who is considered to have no problems taking a drug orally, the prediction device 1 may output a type of drug that corresponds to an oral formulation. On the other hand, for a human subject who is considered to have difficulty taking a drug orally, the prediction device 1 may output types of drugs that correspond to intravenous formulations and subcutaneous formulations. For a human subject at high risk of fracture and progressive symptom of osteoporosis, the prediction device 1 may output a type of drug that is expensive. Examples of drugs that are expensive include parathyroid hormone drugs.

The prediction device 1 may output a type of drug suitable for a human subject based on information regarding diseases, symptoms, and the like for which a drug is contraindicated or unsuitable for the human subject. For example, for a human subject suffering from renal failure, the prediction device 1 does not need to output information regarding drugs such as bisphosphonate preparations and SERM preparations. For example, for a human subject with reduced kidney function, the prediction device 1 does not need to output information regarding active vitamin D3 preparations. Without being limited thereto, the prediction device 1 may present the drug efficacy prediction information even when a drug is contraindicated or unsuitable for the human subject. In this case, the prediction device 1 may explicitly indicate that the drug is contraindicated or unsuitable, and may display this in a manner that is easy for the user to understand. In addition, the prediction device 1 may display the reason why the drug is contraindicated or unsuitable for the human subject in a manner that enables the user to understand.

The prediction device 1 may output a virtual image corresponding to the future bone density, and a virtual image predicted for the human subject's bone. For example, when a plain chest X-ray image of a human subject is used as the subject information, an image corresponding to the future bone density may be reflected in an image of a bone shown in the plain chest X-ray image. The prediction device 1 may be capable of outputting an image and/or a moving image showing a difference (i.e., a change in bone density) between the plain chest X-ray image in the subject information and the virtual image. For example, by having the human subject view such an image and/or a moving image, the human subject can be clearly informed of the human subject's own risk of fracture, the human subject's own risk of developing osteoporosis, and the like. Therefore, even for a human subject who is asymptomatic, the need for countermeasures against the risk of developing a fracture, the risk of developing osteoporosis, and the like can be effectively informed, leading to early intervention.

By outputting at least one of six pieces of information (1) to (6) described above as the drug efficacy prediction information, the prediction device 1 can inform the user whether a drug can be expected to be effective when administered to the human subject. The user who has obtained such drug efficacy prediction information can appropriately consider and select a drug to be administered to the human subject.

### Second Embodiment

### Configuration of Prediction System 100b

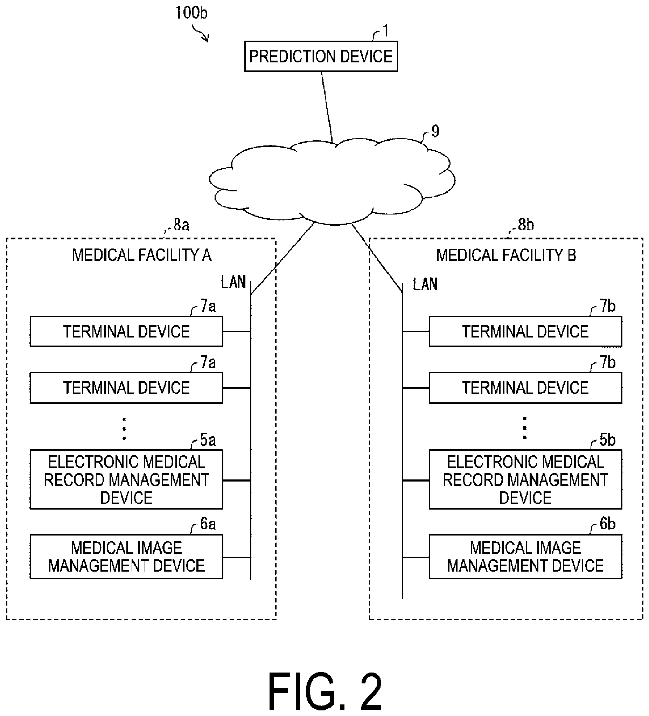

The prediction device 1 need not be a computer installed in the specific medical facility 8, but may be communicably connected to LANs provided in multiple medical facilities 8, respectively, via a communication network 9. FIG. 2 is a diagram illustrating a configuration example of a prediction system 100b according to another aspect of the present disclosure.

In addition to one or more terminal devices 7a (receivers), an electronic medical record management device 5a (storage) and a medical image management device 6a (storage) may be communicably connected to the LAN in a medical facility 8a. In addition to terminal devices 7b (receivers), an electronic medical record management device 5b (storage) and a medical image management device 6b (storage) may be communicably connected to the LAN in a medical facility 8b. In the following, when no particular distinction is made between the medical facilities 8a and 8b, each of them is referred to as a "medical facility 8". Each of the terminal devices 7a and 7b and each of the medical image management devices 6a and 6b are referred to as a "terminal device 7", and a "medical image management device 6", respectively, when distinction is unnecessary.

FIG. 2 illustrates an example in which the LANs in the medical facility 8a and the medical facility 8b are connected to the communication network 9. The prediction device 1 is not limited to the configuration illustrated in FIG. 2, as long as the prediction device 1 is communicably connected to the devices in the medical facilities via the communication network 9. For example, the prediction device 1 may be installed in the medical facility 8a or in the medical facility 8b.

In the prediction system 100b employing such a configuration, the prediction device 1 can acquire a medical image of a human subject Pa examined in the medical facility 8a from the medical image management device 6a in the medical facility 8a. The prediction device 1 then transmits drug efficacy prediction information regarding an effect of a drug that is being considered for administration to the human subject Pa to the terminal device 7a installed in the medical facility 8a. In the same manner as and/or a similar manner to the above, the prediction device 1 can acquire a medical image of a human subject Pb examined in the medical facility 8b and can transmit drug efficacy prediction information regarding an effect of a drug that is considered for administration to the human subject P to the terminal device 7b installed in the medical facility 8b.

In this case, the medical image of each human subject is required to include identification information unique to each medical facility 8 assigned to each medical facility 8 where each human subject is examined (e.g., facility ID), and identification information unique to each human subject assigned to each human subject (e.g., human subject ID, patient ID). Based on these pieces of identification information, the prediction device 1 can correctly transmit drug efficacy prediction information output from the medical image regarding the human subject to the terminal device 7 in each medical facility 8 where the human subject is examined.

### Third Embodiment

### Overview of Prediction Device 1a

Another embodiment of the present disclosure will be described below. For convenience of description, members having the same functions as those of the members described in the above-described embodiment are denoted by the same reference signs, and description thereof is not repeated.

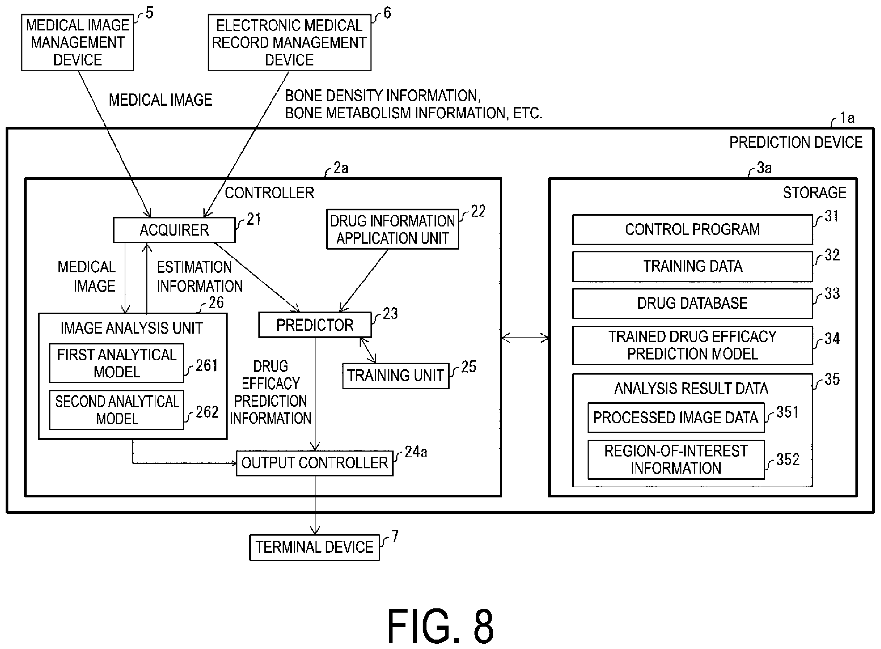

FIG. 8 is a block diagram illustrating a configuration of a prediction device 1a according to the present embodiment. The prediction device 1a is different from the prediction device 1 illustrated in FIG. 3 in that the prediction device 1a further has an image analysis function and a function for generating processed image data 351 in which a medical image is modified so as to include region-of-interest information 352 regarding a region of interest during an image analysis process.

The prediction device 1a includes a controller 2a that collectively controls units in the prediction device 1a, and a storage 3a that stores various data to be used by the controller 2a. The controller 2a includes an acquirer 21, a drug information application unit 22, a predictor 23, an output controller 24a, a training unit 25, and an image analysis unit 26. The storage 3a stores a control program 31, which is a program for performing various controls in the prediction device 1a, as well as a training data 32, a drug database 33, a trained drug efficacy prediction model 34 (drug efficacy prediction model), and analysis result data 35. The analysis result data 35 may store the region-of-interest information 352 output from the image analysis unit 26. The analysis result data 35 stores the processed image data 351 output from the image analysis unit 26. The processed image data 351 is image data in which the image analysis unit 26 adds an analysis result and region-of-interest information to a medical image to be analyzed.

The processed image data 351 may be image data in which information indicating a region serving as a main basis is added to the input image data. The information indicating a region is, for example, information indicating the extent of the region, for example, by coloring or framing. In image analysis, in many cases, a certain region of an image serves as a main basis for estimation, and thus, the user can confirm the region serving as the basis for estimation based on information indicating such a region. In this case, a display image displayed on the terminal device 7 used by the user includes a processed image in which region-of-interest information is added to the medical image input to a first analytical model 261. The output controller 24a may generate a display image in which the medical image and the processed image are placed side by side. With such a display image, the user can easily compare the analyzed original image with the processed image. Alternatively, the terminal device 7 may be capable of separately displaying the medical image and the processed image in response to a user operation. For example, the terminal device 7 may alternately display the medical image and the processed image when the user clicks a switching button displayed on a display screen.

### Image Analysis Unit 26

The image analysis unit 26 includes the first analytical model 261. The first analytical model 261 is, for example, a trained mechanical model that analyzes a medical image, and outputs an estimation result regarding a state change of a bone from an input medical image. In addition to the estimation result, the first analytical model 261 may output the region-of-interest information 352 regarding a region of interest that is a region within the medical image and that is of interest in the process of outputting the estimation result. The image analysis unit 26 may store the region-of-interest information 352 in the storage 3a. Here, the region within the medical image is, for example, a region inside a periphery of the medical image. For example, the region-of-interest information 352 may be located within the entire region in the medical image, or may overlap only a portion in the medical image. Alternatively, when the region of interest is not within the medical image (or is, for example, the entire medical image), the region of interest does not need to overlap with any region within the medical image. In this case, for example, a message indicating that a region of interest is not present (or, for example, a message indicating that the entire medical image is a region of interest) may be displayed outside the medical image.

Alternatively, the region-of-interest information 352 may be a heat map indicating a region on which the estimation result is based. In this case, for example, the outer edge of the heat map indicates the segmented areas. A heat map is a method of representing the magnitude of bone density by the concentration of an arbitrary color. For example, the region-of-interest information 352 may be a heat map indicating a level of interest, or a heat map indicating a numerical value of bone density. The region-of-interest information 352 may be a heat map indicating a likelihood (probability) of fracture. In this case, the processed image may be an image in which a heat map indicating region-of-interest information is superimposed on a medical image. Images used for the heat map may be still images or moving images. By representing a heat map with a moving image, for example, by fading various heat maps in order, the relationship between heat maps can be visually recognized with ease. When the heat map is a heat map of bone density that includes areas other than the segmented areas, a part of the segmented areas may be surrounded by a frame.

A medical image input to the first analytical model 261 is an image including an image of a human subject's bone, and the first analytical model 261 outputs an estimation result regarding a condition of the bone. For example, the first analytical model 261 may be a trained model that has been trained to output an estimation result or a calculation result regarding a bone condition, such as a bone density, relative comparison of bone densities, or a likelihood of fracture, from an X-ray image of the bone. The bone density may be a calculated bone density of a bone site included in the medical image, or may be an average bone density of the whole body estimated from the image data. Alternatively, the bone density at the site with the lowest bone density may be used as the bone density. A known method can be used to calculate the bone density from a medical image. The relative comparison of bone densities is the ratio of estimated bone density with respect to YAM. The likelihood of fracture is a likelihood that a bone will fracture at a particular site (such as the femoral neck). The estimation result regarding the bone condition may be an estimation result at a time when a medical image is captured, or may be prediction at a predetermined time after the medical image is captured.

The first analytical model 261 may output at least one selected from the group consisting of the following pieces of information as an estimation result.
- Bone density estimated at a time when the medical image is captured.
- Bone density predicted at a time a specified period of time after the medical image is captured.
- An estimated fracture site and a likelihood of fracture at a time when the medical image is captured.
- Bone density at a time a specified period of time before the medical image is captured.
- A predicted fracture site and a likelihood of fracture at a time a specified period of time after the medical image is captured.

When the bone density of the subject's bone has not been measured, the acquirer 21 cannot acquire the bone density information of the human subject from the electronic medical record management device 6. In such a case, the acquirer 21 may acquire, from the image analysis unit 26, an estimation result including the bone density of the bone estimated by the first analytical model 261 from a medical image including an image of the bone of the human subject. In this case, the predictor 23 may input the estimation result regarding the bone density acquired from the first analytical model 261 into the trained drug efficacy prediction model 34 as the subject information.

A method of training the first analytical model 261 will be described. In the training of the first analytical model 261, for example, training for estimating a bone density may be performed using training data that includes an X-ray image of a bone with a specified bone density as an explanatory variable, and the bone density of an examinee shown in the X-ray image as a response variable. Training for estimating a likelihood of fracture can be performed using training data that includes an X-ray image showing the examinee's bone as an explanatory variable and data regarding whether the examinee has suffered a fracture within a predetermined period of time as a response variable. The relative comparison of bone densities does not need to be a subject for training and is obtained by dividing the estimated bone density by the YAM.

Training of future prediction may be performed using an X-ray image of a bone with a specified bone density and data on the bone density of the examinee after a predetermined period of time, or data on whether the examinee has suffered a fracture, as teaching data. The data on whether the examinee has suffered a fracture can be, for example, information linking fracture information to an X-ray image showing a fractured bone, and information linking non-fracture information to an X-ray image showing a non-fractured bone. By including in the training data information such as age, sex, weight, and information on lifestyle habits such as an amount of exercise, diet, smoking, and drinking of the examinee serving as the training data, the first analytical model 261 can make more accurate estimation and prediction.

The first analytical model 261 may be a cytopathology analytical model. In this case, the input medical image may be a microscopy image of a human subject's cell, and the estimation result may be information regarding a pathological mutation in the cell.

In the medical image, not only a change in the image due to a change in bone density or the like estimated by the first analytical model, but also a change in the image due to other factors may appear. The other factors include diseases, medical treatment scars, various types of intentionally placed information, and objects worn by the examinee. Examples of diseases include arterial calcification, osteosclerosis, fractures, and organ tumors. Examples of medical treatment scars include implants and bone cement. Examples of intentionally placed information include letters such as "L" and "R" that indicate directions. Examples of objects worn by the examinee include a necklace.

These factors often cause pixel values (e.g., luminance) of a medical image to change compared to when no other factors are present. An implant, a necklace, or the like has a unique shape that may hide information included in the medical image that the first analytical model 261 uses to estimate a bone density. Changes due to these factors may affect the evaluation of bone density based on the estimation result of the image analysis unit 26. Usually, a doctor who views the medical image notices such a change and interprets the result of bone density evaluation in consideration of information that caused the change. However, in some cases, the doctor may overlook such a change in the medical image. Further, there may be cases where the doctor cannot determine how other factors affect the change in the medical image based on the estimation result output from the image analysis unit 26. In such cases, the doctor may be unsure of how to interpret the output estimation result.

Therefore, the image analysis unit 26 may further include a second analytical model 262. The second analytical model 262 is a trained mechanical model that detects changes in a medical image due to other factors, such as diseases, medical treatment scars, various types of intentionally placed information, or objects worn by the examinee. The image analysis unit 26 may correct the medical image according to the detection result. Here, the correction may be at least one selected from the group consisting of image position adjustment (right, left, up, or down direction or rotation relative to the original position), brightness adjustment, and masking. Alternatively, the image analysis unit 26 may analyze the medical image subjected to these corrections using the first analytical model 261.

The second analytical model 262 can be trained using images that have been pre-annotated by a doctor regarding the locations of diseases, medical treatment scars, various types of intentionally placed information, objects worn by the examinee, and the like. The second analytical model 262 can be constructed by a known training method for constructing image analytical models.

The image analysis unit 26 may generate the processed image data 351 using the medical image input to the first analytical model 261, the analysis result and the region-of-interest information output from the first analytical model 261. Alternatively, the image analysis unit 26 may correct an image using a result output from the second analytical model 262, and input the corrected image to the first analytical model 261 to create processed image data. Alternatively, the image analysis unit 26 may generate, as the processed image data 351, image data obtained by adding, to the image data generated using the medical image input to the first analytical model 261, and the analysis result and the region-of-interest information output from the first analytical model 261, information regarding a basis for deriving changes in the image due to other factors (data as a basis for derivation).

For example, when the second analytical model 262 detects changes in the medical image due to other factors, the image analysis unit 26 may generate the processed image data 351 by excluding regions corresponding to the detected changes from the original medical image. The image analysis unit 26 may then input the generated processed image data 351 into the first analytical model 261 to output an estimation result (including a bone density) regarding the condition of the bone shown in the processed image data 251. According to this configuration, the image analysis unit 26 can output an estimation result from a medical image including regions corresponding to changes detected by the second analytical model 262, and an estimation result from a medical image excluding the regions corresponding to the changes detected by the second analytical model 262. By presenting both of these estimation results to the user, the user can evaluate how the regions corresponding to the changes detected by the second analytical model 262 affect the estimation result.

The output controller 24a transmits the drug efficacy prediction information output from the predictor 23 and the processed image data 351 generated by the image analysis unit 26 to the terminal device 7. The output controller 24a may generate a web page using the drug efficacy prediction information output from the predictor 23 and the processed image data 351 generated by the image analysis unit 26. In this case, the output controller 24a is required to transmit to the terminal device 7 access information for accessing the generated web page.

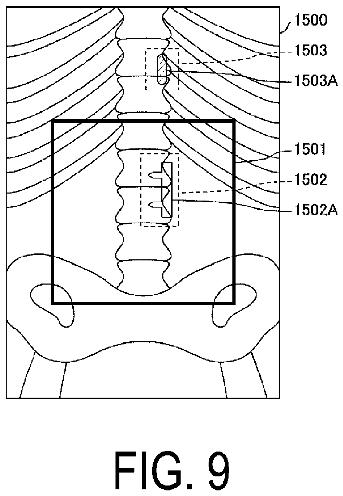

FIG. 9 is an example of a processed image that includes information regarding a basis for deriving changes in an image due to other factors (data as basis for derivation), generated by the image analysis unit 26 based on analysis results analyzed by the first analytical model 261 and the second analytical model 262. A region indicated by a frame 1502 in FIG. 9 is a region in which an implant 1502A that fixes the spine is captured. A region indicated by a frame 1503 is a region in which a calcified abdominal aortic 1503A is captured. In the figure, both regions are indicated by dotted lines, but may be indicated in different colors such as a red frame and a yellow frame. A region indicated by a frame 1501 indicates a region that is a basis for deriving an analysis result analyzed by the first analytical model 261. Thus, a range in which changes in the image due to other factors are detected may be outside the region (frame 1501) that serves as the basis for deriving when the analysis regarding the condition of the object is performed.

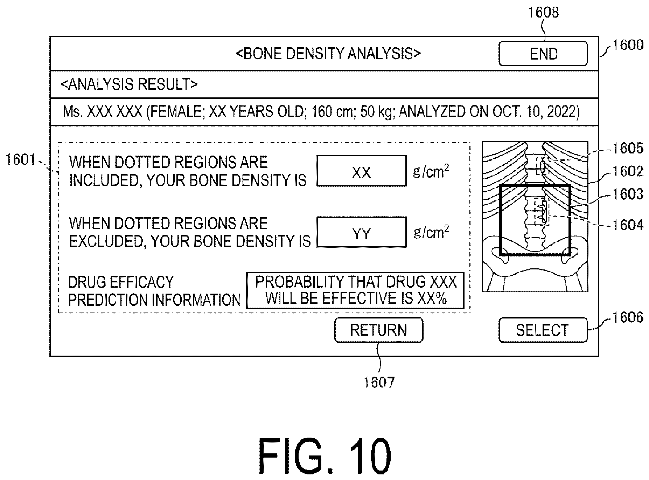

FIG. 10 is an example of a browser image 1600 that displays results of analysis both including and excluding changes in an image due to other factors. The browser image 1600 displays, for example, an analysis image 1602 and an analysis result 1601 including drug efficacy prediction information. The analysis image 1602 may be the same as the image illustrated in FIG. 9.

The analysis result 1601 displays "When the dotted line regions are included, your bone density is XX g/cm²" and "When the dotted line regions are excluded, your bone density is XX g/cm²". The analysis result 1601 illustrated in FIG. 10 also displays drug efficacy prediction information such as "The probability that drug XX is effective is XX%".

In the analysis image 1602, one or both of regions 1604 and 1605 indicated by dotted lines may be selectable by medical personnel such as a doctor. For example, when an operation on a selection button 1606 is received after the region 1604 is selected, a detailed analysis result for the region 1604 may be displayed in the analysis result 1601. A return button 1607 and an end button 1608 are the same as before.

### Fourth Embodiment

Another embodiment of the present disclosure will be described below. For convenience of description, members having the same functions as those of the members described in the above-described embodiment are denoted by the same reference signs, and description thereof is not repeated.

The functions of the prediction devices 1 and 1a, including the drug efficacy prediction model that generates drug efficacy prediction information based on subject information and drug information regarding a drug that has an action on bones, may be enabled by cloud computing. The prediction devices 1 and 1a may be, for example, cloud servers (not illustrated). In this case, in the prediction systems 100 and 100a, the cloud servers may receive subject information from the terminal devices 7, 7a, and 7b, and generate drug efficacy prediction information based on the subject information and the drug information regarding a drug that has an action on bones. The cloud server may then transmit the generated drug efficacy prediction information to the terminal device 7 that is a source of the subject information. In the present embodiment, the terminal devices 7, 7a, and 7b function as storages that store the subject information to be transmitted to the cloud servers, and also function as receivers that receive the drug efficacy prediction information from the cloud servers.

### Example of Implementation by Software

A control block of the prediction device 1 (particularly, the acquirer 21, the drug information application unit 22, the predictor 23, the output controller 24, and the training unit 25) may be implemented by a logic circuit (hardware) formed in an integrated circuit (IC chip) or the like, or may be implemented by software.

In the latter case, the prediction device 1 includes a computer that executes instructions of a program, which is software that implements each function. The computer includes, for example, one or more processors, and also includes a computer-readable recording medium that stores the above program. In the computer, the processor reads the above program from the recording medium and executes the read program to achieve the object of the present disclosure. As the processor, a central processing unit (CPU) can be used, for example. As the recording medium, a "non-transitory tangible medium", for example, a tape, a disk, a card, a semiconductor memory, a programmable logic circuit, or the like can be used in addition to a read only memory (ROM). The computer may further include a random access memory (RAM) for loading the above program. The above program may be supplied to the computer via any transmission medium (communication network, broadcast waves, and the like) that can transmit the program. An aspect of the present disclosure may be implemented in the form of data signals embedded in a carrier wave in which the above program is embodied by electronic transmission.

The invention according to the present disclosure has been described above based on various drawings and examples. However, the invention according to the present disclosure is not limited to the embodiments described above. That is, the invention according to the present disclosure can be modified in various ways within the scope described in the present disclosure, and embodiments obtained by combining technical means disclosed in the different embodiments as appropriate are also included in the technical scope of the invention according to the present disclosure. In other words, it should be noted that a person skilled in the art can easily make various variations or modifications based on the present disclosure. It should also be noted that these variations or modifications are included in the scope of the present disclosure.

### Conclusion 1

According to an aspect 1A of the present disclosure, a prediction device includes an acquirer configured to acquire subject information including at least one selected from the group consisting of fracture risk factor information regarding a fracture risk factor of a subject, bone density information indicating a bone density of a bone of the subject, bone metabolic information indicating a bone metabolic state of the bone of the subject, and a medical image showing the bone of the subject, and a predictor configured to, by inputting the subject information into a drug efficacy prediction model configured to predict an effect of a drug having an action on bones based on drug information regarding the drug, output drug efficacy prediction information regarding an effect of the drug when the drug is administered to the subject.

In a prediction device according to an aspect 2A of the present disclosure, in the aspect 1A, the drug information may include information regarding a drug having an action on bone formation and/or an action on bone resorption.

In a prediction device according to an aspect 3A of the present disclosure, in the aspect 1A or 2A, the drug efficacy prediction model may be trained using teaching data in which multiple pieces of examinee information regarding multiple examinees administered the drug, and the drug information regarding the drug administered to each of the multiple examinees are used as explanatory variables, and an effect of the drug on each of the multiple examinees is used as a response variable.

In a prediction device according to an aspect 4A of the present disclosure, in any one of the aspects 1A to 3A, the drug information may include at least the name of the drug and information regarding a mechanism of an action of the drug.

In a prediction device according to an aspect 5A of the present disclosure, in any one of the aspects 1A to 4A, the drug information may include multiple pieces of information regarding combinations of multiple types of drugs, each of the drugs being able to change a condition of the bone of the subject, and the predictor may output the drug efficacy prediction information for each of the combinations of the multiple types of drugs.

In a prediction device according to an aspect 6A of the present disclosure, in any one of the aspects 1A to 5A, the predictor may output, as the drug efficacy prediction information, information indicating a condition of the bone of the subject after administration of the drug to the subject is continued for a predetermined period of time, or information indicating a change occurring in the bone of the subject before and after the predetermined period of time.

In a prediction device according to an aspect 7A of the present disclosure, in any one of the aspects 1A to 6A, the predictor may output, as the drug efficacy prediction information, information regarding a drug able to change a condition of the bone of the subject after administration to the subject is continued for a predetermined period of time.

In a prediction device according to an aspect 8A of the present disclosure, in any one of the aspects 1A to 7A, the predictor may output a prediction result of future bone density of the bone of the subject as the drug efficacy prediction information.

In a prediction device according to an aspect 9A of the present disclosure, in any one of the aspects 1A to 8A, the predictor may output a prediction result of current bone density of the bone of the subject as the drug efficacy prediction information.

In a prediction device according to an aspect 10A of the present disclosure, in any one of the aspects 1A to 9A, the predictor may output, as the drug efficacy prediction information, a prediction result indicating a difference between a future bone density and a current bone density of the bone of the subject, and/or a prediction estimation result indicating a change in the bone density of the bone of the subject from the current bone density.

In a prediction device according to an aspect 11A of the present disclosure, in any one of the aspects 1A to 10A, the predictor may output a prediction result regarding a fracture site of the subject as the drug efficacy prediction information.

In a prediction device according to an aspect 12A of the present disclosure, in any one of the aspects 1A to 11A, the predictor may output a prediction result indicating a likelihood that the subject suffers a fracture in the future as the drug efficacy prediction information.

In a prediction device according to an aspect 13A of the present disclosure, in any one of the aspects 1A to 12A, the subject information may include at least one selected from the group consisting of the following information regarding the subject: type, age, sex, weight, height, presence or absence of a fracture, fracture site, fracture history, fracture history of family members, glucocorticoids, rheumatoid arthritis, secondary osteoporosis, underlying diseases, smoking history, drinking habits, occupational history, exercise history, medical history, blood test results, urine test results, drugs being taken, and gene sequence.

In a prediction device according to an aspect 14A of the present disclosure, in any one of the aspects 1A to 13A, the bone density information may be a measured value of bone density of a bone of the subject at a predetermined site.

In a prediction device according to an aspect 15A of the present disclosure, in any one of the aspects 1A to 14A, the bone metabolic information may include at least one selected from the group consisting of bone formation marker information regarding osteoblasts, bone resorption marker information regarding osteoclasts, and bone matrix-related marker information regarding the bone quality of the subject.

In a prediction device according to an aspect 16A of the present disclosure, in the aspect 15A, the bone formation marker information may be information regarding alkaline phosphatase and/or procollagen type I N-propeptide, the bone resorption marker information may be information regarding at least one selected from the group consisting of deoxypyridinoline, type I collagen cross-linked N-telopeptide, type I collagen cross-linked C-telopeptide, and tartrate-resistant acid phosphatase-5b fraction (TRACP-5b), and the bone matrix-related marker information may be information regarding at least one selected from the group consisting of undercarboxylated osteocalcin, pentosidine, and homocysteine.

In a prediction device according to an aspect 17A of the present disclosure, in any one of the aspects 1A to 16A, the medical image may include at least one selected from the group consisting of an X-ray image, a computed tomography (CT) image, a magnetic resonance imaging (MRI) image, a positron emission tomography (PET) image, and an ultrasonic image of a body of the subject.

According to an aspect 18A of the present disclosure, a prediction system includes the prediction device according to any one of the aspects 1A to 17A, and a terminal device communicably connected to the prediction device, the terminal device being configured to present the drug efficacy prediction information.

According to an aspect 19A of the present disclosure, a prediction method includes: acquiring subject information including at least one selected from the group consisting of fracture risk factor information regarding a fracture risk factor of a subject, bone density information indicating a bone density of a bone of the subject, bone metabolic information indicating a bone metabolic state of the bone of the subject, and a medical image showing the bone of the subject, and predicting and outputting drug efficacy prediction information regarding an effect of a drug having an action on bones when the drug is administered to the subject by inputting the subject information into a drug efficacy prediction model configured to predict an effect of the drug based on drug information regarding the drug.

According to an aspect 20A of the present disclosure, a control program is a control program for causing a computer to function as the prediction device according to any one of the aspects 1A to 17A, the control program causing the computer to function as the acquirer and the predictor.

According to an aspect 21A of the present disclosure, a recording medium is a computer-readable recording medium recording the control program according to the aspect 20A.

### Conclusion 2

According to an aspect 1B of the present disclosure, a prediction system includes a storage configured to store subject information regarding a bone of a subject, and a receiver configured to receive drug efficacy prediction information regarding an effect of a drug when the drug is administered to the subject, in which the subject information includes at least a medical image showing the bone of the subject, and the prediction system generates the drug efficacy prediction information based on a drug efficacy prediction model configured to predict an effect of a drug having an action on bones based on the subject information and drug information regarding the drug.

In a prediction system according to an aspect 2B of the present disclosure, in the aspect 1B, the drug efficacy prediction model is trained using training data in which multiple pieces of examinee information regarding multiple examinees administered the drug, and the drug information regarding the drug administered to each of the multiple examinees are used as explanatory variables, and an effect of the drug on each of the multiple examinees is used as a response variable.

In a prediction system according to an aspect 3B of the present disclosure, in the aspect 1B or 2B, the drug efficacy prediction information includes at least one selected from the group consisting of a prediction result indicating a difference between a future bone density and a current bone density of the bone of the subject, a prediction result indicating a change in bone density of the bone of the subject from the current bone density, a prediction result when a drug is administered and a prediction result when the drug is not administered, and a prediction result when a different type of drug is administered.

In a prediction system according to an aspect 4B of the present disclosure, in any one of the aspects 1B to 3B, the subject information includes at least one selected from the group consisting of fracture risk factor information regarding a fracture risk factor of the subject, bone density information indicating a bone density of a bone of the subject, and bone metabolic information indicating a bone metabolic state of the bone of the subject.

In a prediction system according to an aspect 5B of the present disclosure, in any one of the aspects 1B to 4B, the drug information includes information regarding a drug having an action on bone formation and/or an action on bone resorption.

In a prediction system according to an aspect 6B of the present disclosure, in any one of the aspects 1B to 5B, the drug information includes at least a name of the drug and/or information regarding a mechanism of an action of the drug.

In a prediction system according to an aspect 7B of the present disclosure, in any one of the aspects 1B to 6B, the drug information may include multiple pieces of information regarding combinations of multiple types of drugs, each of the drugs being able to change a condition of the bone of the subject, and the drug efficacy prediction information may include multiple pieces of information regarding the combinations of the multiple types of drugs.

In a prediction system according to an aspect 8B of the present disclosure, in any one of the aspects 1B to 7B, the drug efficacy prediction information includes information indicating a condition of the bone of the subject after administration of the drug to the subject is continued for a predetermined period of time, or information indicating a change occurring in the bone of the subject before and after the predetermined period of time.

In a prediction system according to an aspect 9B of the present disclosure, in any one of the aspects 1B to 8B, the drug efficacy prediction information includes information regarding a drug able to change a condition of the bone of the subject after administration to the subject is continued for a predetermined period of time.

In a prediction system according to an aspect 10B of the present disclosure, in any one of the aspects 1B to 9B, the drug efficacy prediction information includes a prediction result of future bone density of the bone of the subject.

In a prediction system according to an aspect 11B of the present disclosure, in any one of the aspects 1B to 10B, the drug efficacy prediction information includes a prediction result of current bone density of the bone of the subject.

In a prediction system according to an aspect 12B of the present disclosure, in any one of the aspects 1B to 11B, the drug efficacy prediction information includes a prediction result regarding a fracture site of the subject.

In a prediction system according to an aspect 13B of the present disclosure, in any one of the aspects 1B to 12B, the drug efficacy prediction information includes a prediction result indicating a likelihood that the subject suffers a fracture in the future.

In a prediction system according to an aspect 14B of the present disclosure, in any one of the aspects 1B to 13B, the subject information includes at least one selected from the group consisting of fracture risk factor information regarding a fracture risk factor of the subject, bone density information indicating a bone density of a bone of the subject, and bone metabolic information indicating a bone metabolic state of the bone of the subject, and the bone density information is a measured value of the bone density of a bone of the subject at a predetermined site.

In a prediction system according to an aspect 15B of the present disclosure, in any one of the aspects 1B to 14B, the subject information includes at least one selected from the group consisting of fracture risk factor information regarding a fracture risk factor of the subject, bone density information indicating a bone density of a bone of the subject, and bone metabolic information indicating a bone metabolic state of the bone of the subject, the medical image is a plain X-ray image of a body of the subject, and the bone density information is an estimated value estimated based on a bone density prediction model configured to estimate a bone density of the bone from the plain X-ray image showing the subject.

In a prediction system according to an aspect 16B of the present disclosure, in the aspect 15B, the bone density prediction model is trained using teaching data in which a medical image showing a bone of an examinee is used as an explanatory variable, and a measured value of bone density of the bone of the examinee shown in the medical image is used as a response variable.

According to an aspect 17B of the present disclosure, a prediction device includes an acquirer configured to acquire subject information regarding a bone of a subject, and a predictor configured to output drug efficacy prediction information regarding an effect of a drug when the drug is administered to the subject, in which the subject information includes at least a medical image showing the bone of the subject, and the predictor includes a drug efficacy prediction model configured to predict an effect of a drug having an action on bones based on the subject information and drug information regarding the drug.

According to an aspect 18B of the present disclosure, a prediction method includes storing subject information regarding a bone of a subject, generating drug efficacy prediction information regarding an effect of a drug having an action on bones when the drug is administered to the subject based on a drug efficacy prediction model configured to predict the effect of the drug based on the subject information and drug information regarding the drug, and receiving the drug efficacy prediction information, in which the subject information includes at least a medical image showing the bone of the subject.

According to an aspect 19B of the present disclosure, a control program is a control program for causing a computer to function as the prediction system according to any one of the aspects 1B to 16B, the control program causing the computer to function as the storage and the receiver.

According to an aspect 20B of the present disclosure, a recording medium is a computer-readable recording medium recording the control program according to the aspect 19B.

### REFERENCE SIGNS

1, 1a Prediction device
5 Medical image management device (storage)
6 Electronic medical record management device (storage)
7 Terminal device (storage, receiver)
21 Acquirer
22 Drug information application unit
23 Predictor
24 Output controller
25 Training unit
32 Teaching data
34 Trained drug efficacy prediction model
100a, 100b Prediction system
S11 Acquiring step
S12 Generating step

## Claims

1. A prediction system comprising:
a storage configured to store subject information regarding a bone of a subject; and
a receiver configured to receive drug efficacy prediction information regarding an effect of a drug when the drug is administered to the subject, wherein
the subject information comprises at least a medical image showing the bone of the subject, and
the prediction system generates the drug efficacy prediction information based on a drug efficacy prediction model configured to predict an effect of a drug having an action on bones based on the subject information and drug information regarding the drug.

2. The prediction system according to claim 1, wherein
the drug efficacy prediction model is trained using training data in which multiple pieces of examinee information regarding multiple examinees administered the drug, and the drug information regarding the drug administered to each of the multiple examinees are used as explanatory variables, and an effect of the drug on each of the multiple examinees is used as a response variable.

3. The prediction system according to claim 1 or 2, wherein
the drug efficacy prediction information comprises at least one selected from the group consisting of a prediction result indicating a difference between a future bone density and a current bone density of the bone of the subject, a prediction result indicating a change in bone density of the bone of the subject from the current bone density, a prediction result when a drug is administered and a prediction result when the drug is not administered, and a prediction result when a different type of drug is administered.

4. The prediction system according to any one of claims 1 to 3, wherein
the subject information comprises at least one selected from the group consisting of fracture risk factor information regarding a fracture risk factor of a subject, bone density information indicating a bone density of a bone of the subject, and bone metabolic information indicating a bone metabolic state of the bone of the subject.

5. The prediction system according to any one of claims 1 to 4, wherein
the drug information comprises information regarding a drug having an action on bone formation and/or an action on bone resorption.

6. The prediction system according to any one of claims 1 to 5, wherein
the drug information comprises at least a name of the drug and/or information regarding a mechanism of an action of the drug.

7. The prediction system according to any one of claims 1 to 6, wherein
the drug information comprises multiple pieces of information regarding combinations of multiple types of drugs, each of the drugs being able to change a condition of the bone of the subject, and
the drug efficacy prediction information comprises multiple pieces of information regarding the combinations of the multiple types of drugs.

8. The prediction system according to any one of claims 1 to 7, wherein
the drug efficacy prediction information comprises information indicating a condition of the bone of the subject after administration of the drug to the subject is continued for a predetermined period of time, or information indicating a change occurring in the bone of the subject before and after the predetermined period of time.

9. The prediction system according to any one of claims 1 to 8, wherein
the drug efficacy prediction information comprises information regarding a drug able to change a condition of the bone of the subject after administration to the subject is continued for a predetermined period of time.

10. The prediction system according to any one of claims 1 to 9, wherein
the drug efficacy prediction information comprises a prediction result of future bone density of the bone of the subject.

11. The prediction system according to any one of claims 1 to 10, wherein
the drug efficacy prediction information comprises a prediction result of current bone density of the bone of the subject.

12. The prediction system according to any one of claims 1 to 11, wherein
the drug efficacy prediction information comprises a prediction result regarding a fracture site of the subject.

13. The prediction system according to any one of claims 1 to 12, wherein
the drug efficacy prediction information comprises a prediction result indicating a likelihood that the subject suffers a fracture in the future.

14. The prediction system according to any one of claims 1 to 13, wherein
the subject information comprises at least one selected from the group consisting of fracture risk factor information regarding a fracture risk factor of a subject, bone density information indicating a bone density of a bone of the subject, and bone metabolic information indicating a bone metabolic state of the bone of the subject, and
the bone density information is a measured value of bone density of a bone of the subject at a predetermined site.

15. The prediction system according to any one of claims 1 to 14, wherein
the subject information comprises at least one selected from the group consisting of fracture risk factor information regarding a fracture risk factor of a subject, bone density information indicating a bone density of a bone of the subject, and bone metabolic information indicating a bone metabolic state of the bone of the subject,
the medical image is a plain X-ray image of a body of the subject, and
the bone density information is an estimated value estimated based on a bone density prediction model configured to estimate a bone density of the bone from the plain X-ray image showing the subject.

16. The prediction system according to claim 15, wherein
the bone density prediction model is trained using teaching data in which a medical image showing a bone of an examinee is used as an explanatory variable, and a measured value of bone density of the bone of the examinee shown in the medical image is used as a response variable.

17. A prediction device comprising:
an acquirer configured to acquire subject information regarding a bone of a subject; and
a predictor configured to output drug efficacy prediction information regarding an effect of a drug when the drug is administered to the subject, wherein
the subject information comprises at least a medical image showing the bone of the subject, and
the predictor comprises a drug efficacy prediction model configured to predict an effect of a drug having an action on bones based on the subject information and drug information regarding the drug.

18. A prediction method comprising:
storing subject information regarding a bone of a subject;
generating drug efficacy prediction information regarding an effect of a drug having an action on bones when the drug is administered to the subject based on a drug efficacy prediction model configured to predict the effect of the drug based on the subject information and drug information regarding the drug; and
receiving the drug efficacy prediction information, wherein
the subject information comprises at least a medical image showing the bone of the subject.

19. A control program for causing a computer to function as the prediction system according to any one of claims 1 to 16, the control program causing the computer to function as the storage and the receiver.

20. A computer-readable recording medium recording the control program according to claim 19.
